# EUROPÄISCHE PATENTANMELDUNG

(11) **EP 3 804 704 A1**
(43) Veröffentlichungstag der Anmeldung: **14.04.2021**
(21) Anmeldenummer: 19202462.8
(22) Anmeldetag: 10.10.2019
(51) Int. Cl.: A61K 9/51, A61K 31/5377

(54) **VERFAHREN ZUR HERSTELLUNG NANOPARTIKULÄREN RIVAROXABANS**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein Verfahren zur Herstellung nanopartikulären Rivaroxabans, umfassend die Schritte: a) Bereitstellen einer Lösung von Rivaroxaban in einem Lösungsmittel, Bereitstellen eines Anti-Lösungsmittels für das Rivaroxaban und Bereitstellen von mindestens einem Stabilisator, wobei der Stabilisator in dem Lösungsmittel und/oder dem Anti-Lösungsmittel gelöst vorliegt und b) Mischen der Lösung des Rivaroxabans in dem Lösungsmittel, des Antilösungsmittels und des Stabilisators in einem Mikromischer unter Erhalt einer Suspension aufweisend ausgefälltes Rivaroxaban, das Lösungsmittel und das Anti-Lösungsmittel, wobei das ausgefällte Rivaroxaban in Form von Nanoteilchen vorliegt. Nach Schritt b) wird der Schritt c) durchgeführt: c) Entfernen des Lösungsmittels und des Anti-Lösungsmittels unter Erhalt von Aggregaten aufweisend das nanopartikuläre Rivaroxaban.

## Beschreibung

Die vorliegende Erfindung betrifft ein Verfahren zur Herstellung nanopartikulären Rivaroxabans, nanopartikuläres Rivaroxaban und eine Wirkstoffformulierung aufweisend nanopartikuläres Rivaroxaban.

Ein hoher Anteil pharmazeutischer Wirkstoffe von über 40 % sind der BCS (Biopharmaceutics Classification System) Klasse 2 bzw. 4 zuzuordnen. Die Wirkstoffe in diesen Klasse verfügen über eine schlechte Löslichkeit und hohe Bioverfügbarkeit bzw. schlechte Löslichkeit und schlechte Bioverfügbarkeit. Um die schlechte Löslichkeit zu verbessern ist es eine allgemein akzeptierte Methode die Partikelgröße der Wirkstoffe zu reduzieren und so ein großes Oberflächen-zu-Volumenverhältnis zu schaffen. Entsprechend der Noyes-Whitney-Gleichung wird so eine hohe Löslichkeit der Partikelgrenzschicht geschaffen.

Bekannte Top-down Methoden wie zum Beispiel die Nanomahlung oder die Hochdruckhomogenisation werden standardmäßig zur Herstellung von Mikro- und Nano-Wirkstoffpartikeln genutzt. Jedoch weisen diese Methoden einige Limitierungen auf, wie zum Beispiel ein hoher Energieeintrag, geringe Ausbeuten, Kontamination durch Abrieb, und schwer kontrollierbare Partikelgrößen und Oberflächeneigenschaften, welche ihre Anwendung und weitere Kommerzialisierung erschwert.

Der dieser Erfindung zu Grunde liegende Fällungsprozess (Bottom-up Methode) ist eine dem Fachmann bekannte Standardmethode um Mikro- und Nanopartikel herzustellen. Entsprechend der Vorarbeiten anderer Wissenschaftler ist bekannt, dass ein Reaktor mit exzellenter Mischqualität entscheidend für die gleichmäßige Herstellung von Wirkstoff-Nanopartikeln ist. Ein Mikrokanalreaktor bietet ein definiertes Reaktionsvolumen und einen definierten Kanal, welcher in Hinsicht auf Konzentration (Stofftransport) und Temperatur (Wärmetransport) zu einer besseren Reaktionskontrolle führt. Der Fällungsschritt selbst wird im Mikrokanalreaktor durch eine schnelle Vermischung, die zu einer schnellen Übersättigung führt, kontrolliert. Die Übersättigung wiederum bestimmt die Partikelgröße und ihre Verteilung, wie zum Beispiel Keimbildungsrate und Keimwachstum.

Die Kombination aus Fällung und Mikrokanalreaktor um Mikro- und Nanopartikeln herzustellen wurden in zahlreichen Publikationen und Patenten beschrieben. Die Partikelgrößen bewegen sich dabei in Bereichen zwischen 100 nm und 80 µm.

US 2013/0012551 A1 beschreibt eine Methode zur Herstellung von Mikro- und Nanopartikeln aus wasserlöslichen und wasser-unlöslichen Substanzen durch die Fällung in einem Mikrojet Reaktor. Dabei werden Solvent, enthaltend das zu fällende Produkt, und ein Antisolvent in Form von Jets in einem Mikrojet-Reaktor bei definierten Drücken und Volumenströmen gemischt, um eine schnelle Fällung, Co-Fällung oder eine chemische Reaktion zu beeinflussen, bei der Mikro- oder Nanopartikeln entstehen. Die Partikelgröße wird dabei über die Temperatur im System, den Volumenströmen des Solvent und Antisolvent und/oder des Gases kontrolliert. Kleinere Partikelgrößen werden dabei bei niedrigen Temperaturen, bei hohen Solvent und Antisolvent Volumenströmen und/oder vollständigen Abwesenheit des Gasstroms erzielt. In der Methode wird nur ein Mikrojet Reaktor zur Fällung genutzt. Die Partikelgrößen liegen im Bereich zwischen 141,2-358 nm. Nachteilig ist bei dieser Methode, dass die Partikelgrößenstabilität, insbesondere die Langzeitstabilität nicht behandelt wird.

Hong Zhao et al., Ind. Eng. Chem. Res. 2007, 46, 8229 - 8235 beschreibt eine Solvent Antisolvent Fällung (LSAP) in einem Mikrokanalreaktor. Die Partikelgröße wurde dabei von 55 µm zu 364 nm reduziert. Nachteilig bei dieser Methode ist die Filtrierung der Probe nach der Fällung mit einem 0,45 µm Filter. Nachteilig ist bei dieser Methode zudem, dass die Partikelgrößenstabilität, insbesondere die Langzeitstabilität nicht behandelt wird.

Yuancai Dong et al., Powder Technology 2014, 268, 424-428, kombinieren Antisolvent-Fällung in einem Mikrokanalreaktor mit Sprühtrocknung. Es werden Partikelgrößen zwischen 196-296 nm erzielt. Die Gesamtvolumenströme liegen maximal bei 4 ml/min. Nachteilig ist, dass der Prozess für den Industriellen Scale-Up nicht geeignet ist. Nachteilig ist auch, dass obwohl die Redispergierung in einer Wasser-SDS Lösung erfolgte, keine nahezu vollständige Redispergierung möglich ist. Die redispergierte Suspension weist mittlere Partikelgrößen um die 350 nm auf.

Jiahui Hu et al European Journal of Pharmaceutical Sciences 2003, 20, 295-303, entwickelten die Sprühgefrierung in Flüssigkeiten (SFL Technologie) um mikronisierte Pulver zu erhalten. Dabei wird eine Wirkstofflösung direkt in eine kryogenen Flüssigkeit eingesprüht um gefrorene nanostrukturierte Partikel zu erhalten. Diese werden anschließend mittels Gefriertrocknung getrocknet. Dabei werden bei der Partikelgrößenverteilung D(10)-Werte bei 0,14 µm, D(50)-Werte bei 0,68 µm und D(90)-Werte bei 15,89 µm erzielt. Nachteilig ist, dass bei diesem Prozess die Polydispersität der hergestellten Partikel sehr breit ist. Zudem müssen kritische Lösemittel wie Acetonitrile oder THF zur Lösung des Wirkstoffs verwendet werden. Der Wirkstoffgehalt in der Ursprungslösung liegt bei maximal 2,2 Gew.-%.

Niva et al (2013) kombinieren die Nassmahlung mit der Sprüh-Gefriertrocknung. Als Stabilisatoren werden PVP (Polymer) und SLS (Tensid) genutzt. Die Partikelgrößen nach der Mahlung liegen zwischen 170-180 nm. Nachteilig ist, dass die redispergierten Partikel bei 1-100 µm liegen.

Mahesh V. Chaubal Pharmaceutical Research, 2008, 25, 10, berichtet von redispergierbaren nanopartikelenthaltenden Pulvern und der Wichtigkeit von geladenen Tensiden in Bezug auf die Stabilität von Partikeln während der Trocknung. Nachteilig an dieser Methode ist, dass die durchschnittliche Partikelgröße der Suspensionen nach der Redispergierung stets 10-20% größer ist als im Vergleich zu den Ursprungssuspensionen vor der Trocknung. Des Weiteren werden zusätzlich zu den Stabilisierungsadditiven (Poloxamer 188 und Natriumdesoxycholat) Gerüstbildner wie Lactose, Saccharose und Mannitol verwendet. Zusätzlich sind die Partikeln wesentlich gröber (99% < 1µm) (auch vor der Trocknung (99%<0,8µm).

Andrej Dolenc International Journal of Pharmaceutics, 2009, 376, 204-212 et al, stellt Nano-Suspensionen mit PVP und SDS als Additive her und trocknet diese mittels Sprühtrocknung. Nachteilig an dieser Methode ist, dass die Pulver zwar redispergierbar sind, allerdings sind deutliche Abweichungen im D(90)-Wert (30%) zwischen der Ursprungs- und redispergierten Suspension zu erkennen. Die möglichen Vorteile von ionischen Tensiden für die Stabilität der Nanopartikeln während der Trocknung sind nicht beschrieben. SDS fungiert nur als Additiv für die Bildung stabiler Nanopartikel mittels Fällung. Zusätzlich liegen wesentlich gröbere Partikeln im D(90)-Bereich vor (>1µm).

Xu Xue et al., AAPS PharmSciTech, 2018, 19, 4 beschreiben die Herstellung und Optimierung von Rivaroxaban Freisetzung durch Selbst-nanoemulgierende Wirkstofffreisetzungssysteme (SNEDDS) für eine verbesserte orale Bioverfügbarkeit. Hierbei werden Tröpfechengrößen < 100 nm erreicht.

US 2015/0335753 A1 beschreibt die Herstellung von schwer löslichen Wirkstoffen mit mittleren Partikelgrößen < 1 µm bis < 500 nm. Die Formulierung beinhaltet dabei einen aktiven Wirkstoff, ein wasserlösliches Tensid, und ein wasserlösliches Polymer und wird durch eine fixe Geometrie wie dem "MICROFLUIDIZER" der Firma Microfluidics Corporation of Newton, Mass. USA gefördert. Der "MICROFLUIDIZER" zwingt die Suspension unter hohen Druck durch Mikrokanäle in eine Kammer, wobei zwei entgegensetzte Suspensionströme miteinander kollidieren und anschließend senkrecht zur Kollisionsebene herausgefördert werden. Der Auslassstrom kann rezirkuliert werden bis die gewünschte Partikelgröße erreicht ist. Die Erfindung beinhaltet auch die Weiterverarbeitung der Suspension durch Entfernung des wässrigen Lösemittels und Erhalt des Wirkstoffs als getrocknetes Pulver.

Michael E. Matteucci et al., Langmuir, 2006, 22, 8951-8959 fällen den praktisch unlöslichen Wirkstoff Itraconazol durch eine Solvent Antisolventfällung auf D(90)-Werte von 370 nm. Die Solvent-Lösung besteht dabei aus Itraconazol und Poloxamer 407 in Tetrahydrofuran (THF). THF ist als Gefahrstoff vermutlich krebserregend.

Rivaroxaban ist ein pharmazeutischer Wirkstoff mit schlechter Löslichkeit. Es wird als Blutgerinnungshemmer (Antikoagulanz) verwendet und ist auch unter dem chemischen Namen (S)-5-Chlor-N-{2-oxo-3-[4-(3-oxomorpholin-4-yl)phenyl]-1,3-oxazolidin-5-ylmethyl}thiophen-2-carbamid bekannt. Rivaroxaban ist im Arzneimittel Xarelto als Arzneistoff enthalten.

Die vorliegende Erfindung hat sich die Aufgabe gestellt, ein Verfahren zur Herstellung von gut redispiergierbarem, nanopartikulärem Rivaroxaban, bereitzustellen. Zudem soll gut redispergierbares, nanopartikuläres Rivaroxaban und eine Wirkstoffformulierung aufweisend gut redispergierbares, nanopartikuläres Rivaroxaban bereitgestellt werden.

Erfindungsgemäß gelöst wird diese Aufgabe durch ein Verfahren gemäß Anspruch 1, die Partikel nach Anspruch 14 und die Formulierung nach Anspruch 15. Vorteilhafte Weiterbildungen sind in den Unteransprüchen angegeben. Sie können beliebig kombiniert werden, sofern sich aus dem Kontext nicht eindeutig das Gegenteil ergibt.

Mit der Erfindung wird ein Verfahren zur Herstellung nanopartikulären Rivaroxabans vorgeschlagen.

Das Verfahren umfasst die Schritte:
a) Bereitstellen einer Lösung von Rivaroxaban in einem Lösungsmittel, Bereitstellen eines Anti-Lösungsmittels für das Rivaroxaban und Bereitstellen von mindestens einem Stabilisator, wobei der Stabilisator in dem Lösungsmittel und/oder dem Anti-Lösungsmittel gelöst vorliegt und
b) Mischen der Lösung des Rivaroxabans in dem Lösungsmittel, des Antilösungsmittels und des Stabilisators in einem Mikromischer unter Erhalt einer Suspension aufweisend ausgefälltes Rivaroxaban, das Lösungsmittel und das Anti-Lösungsmittel,
wobei das ausgefällte Rivaroxaban in Form von Nanoteilchen vorliegt,
wobei nach Schritt b) der Schritt c) durchgeführt wird:
c) Entfernen des Lösungsmittels und des Anti-Lösungsmittels unter Erhalt von Aggregaten aufweisend das nanopartikuläre Rivaroxaban.

Es konnte in überraschender Weise gezeigt werden, dass mit diesem Verfahren eine Suspension mit ausgefälltem Rivaroxaban in Form von Nanoteilchen mit kleiner Partikelgröße und enger Partikelgrößenverteilung erhalten werden kann. Es kann zudem eine Stabilisierung der Partikelgröße direkt nach der Fällung erreicht werden, die Langzeitstabilität der RivaroxabanPartikel kann verbessert werden und es kann sich eine verbesserte Handhabung von Aggregaten, die das nanopartikuläre Rivaroxaban aufweisen, für die Weiterverarbeitung in oralen und parenteralen Darreichungsformen ergeben.

Ein Merkmal mikrostrukturierter Bauteile wie Mikromischern sind die kleinen Abmessungen der Fluidkanäle, die typischerweise im Bereich zwischen 10 und 5000 µm angesiedelt sind. Aus diesem Grund können beispielsweise mit Multilaminationsmischern feine Fluidlamellen erzeugt werden, zwischen denen auf Grund ihrer geringen Dicke ein schneller Stoffaustausch durch Diffusion erfolgen kann. Vorzugsweise enthalten die Mikromischer Mischplatten mir Nenndurchmessern der Schlitze zwischen 100 und 400 µm.

Mikromischer im erfindungsgemäßen Verfahren sind Mischer zum Vermischen von mindestens zwei Fluidströmen, in denen interne Leitungen Durchmesser von weniger als einem Millimeter aufweisen. Ein- oder mehr Zentrifugalpumpen, ein in-line Homogenisator, ein Ultraschallmischer, ein Mikromischer und weitere Kombinationen von solchen Mischern können auch verwendet werden, im Besonderen in den Fällen, bei denen es erwünscht ist, die Verweilzeit in der Mischzone zu erhöhen. Bevorzugte Mischer sind Mikrokanalreaktoren wie Ventilmikromischer, Kaskadenmikromischer und LH Typ Mikromischer. Diese Mikrokanalreaktoren bieten verbesserte Mikrovermischungseffekte und dadurch eine enge Partikelgrößenverteilung und kleinere Partikelgrößen.

In einer Ausführungsform des Verfahrens ist der Mikromischer ein Ventilmischer oder ein Kaskadenmischer.

In einem Ventilmischer kann eine Rückströmsperre das Zurückströmen der Mischung in Zuführleitungen fast vollständig oder vollständig verhindern. Bevorzugte Ventilmikromischer sind solche mit einem ersten Kanal für die Zufuhr eines ersten Teilstroms und mit einem zweiten Kanal für die Zufuhr eines zweiten Teilstroms, die in flachen Eintrittsspalten in eine Misch- und Reaktionszone münden und die Misch- und Reaktionszone über einen Auslasskanal verlassen, wobei zwischen der Misch- und Reaktionszone und mindestens einem Kanal für die Zufuhr eines Teilstroms eine Rückströmsperre angeordnet ist. Einer der Zufuhrkanäle weist vorteilhafterweise eine Rückströmsperre in dem Abschnitt auf, in dem der Zufuhrkanal sich zur Misch- und Reaktionszone aufweitet. Solche Mikromischer sind unter anderem in WO 2005/079964 A1 beschrieben.

Das Mischprinzip von Kaskadenmischern beruht auf der sogenannten split-and-recombine-Operation. Bevorzugt ist hier ein statischer Mikrovermischer mit Zuführkammern für mindestens zwei zu vermischende Fluide, von denen Mikrokanäle zu einer Mischkammer führen, wobei die Mikrokanäle in mindestens zwei aneinander liegenden Zuführelementen angeordnet sind, wobei die Zuführelemente keilförmige Platten sind, die mindestens zu einem Ringsektor zusammensetzbar sind, der die Mischkammer bogenförmig umgibt, und wobei die für jedes Fluid vorgesehenen Mikrokanäle eine symmetrische, mindestens zwei Stufen umfassende Bifurkationskaskade bilden. Solche Mikromischer sind unter anderem in WO 2001/043857 A1 beschrieben.

Das erfindungsgemäße Verfahren wird vorzugsweise als kontinuierliches Verfahren durchgeführt. Dann hat es den Vorteil eines einstufigen kontinuierlichen Prozesses basierend auf dem homogenen Keimbildungsmechanismus. Dieser liefert in einem effizienten Prozess ein gleichmäßigeres Produkt und eine kleinere Partikelgröße. Zudem ermöglicht es die Einstellung der Partikelgröße durch Anpassung weniger Parameter, wie beispielsweise dem Solvent zu Antisolvent Verhältnis.

Das erfindungsgemäße Verfahren kann mit kurzen Verweilzeiten im Mikromischer durchgeführt werden ohne weiteren Energieeintrag und bei Atmosphärendruck. Die Verweilzeiten können beispielsweise im Bereich von ≥ 0.01 Sekunden bis ≤ 0.4 Sekunden liegen.

Im erfindungsgemäßen Verfahren kann nanopartikuläres Rivaroxaban mittels einer kontinuierlichen Fällungsmethode über eine Mikroreaktionstechnologie hergestellt werden. Das zu fällende Rivaroxaban ist dabei in einem Lösemittel (nachfolgend auch "Solvent" genannt), in dem es gut löslich ist, gelöst. Ein weiteres Lösemittel, welches bevorzugt vollständig mit dem Solvent mischbar ist, wird mit dem Solvent vermischt. Dieses wird als Anti-Lösungsmittel oder Anti-Solvent bezeichnet. Das zu fällende Rivaroxaban ist dabei schwer löslich im Anti-Solvent und wird durch lokale Übersättigung zur Keimbildung getrieben. Die Keime wachsen zu Partikeln an.

Die Rivaroxabankonzentration in der Lösung ist bevorzugt nahe der praktischen Löslichkeitsgrenze des Lösemittels.

Solche Konzentrationen hängen vom ausgewählten Lösemittel ab, aber sind typischerweise im Bereich von 0.1 bis 20 Gewichts-%. Bevorzugt ist die Rivaroxabankonzentration in der Lösung im Bereich von ≥ 1 bis ≤ 8 Gewichts-%, besonders bevorzugt von ≥ 4 bis ≤ 5 Gewichts-%.

In einer bevorzugten Ausgestaltung der Erfindung kann vorgesehen sein, dass die in Schritt b) ausgefällten Nanoteilchen eine durchschnittliche Teilchengröße, bestimmt mittels Laserdiffraktometrie (LD) oder dynamischer Lichtstreuung (DLS) gemäß ISO 13320, von ≥ 20 nm bis ≤ 900 nm aufweisen.

Besonders bevorzugt weisen die in Schritt b) ausgefällten Nanoteilchen eine durchschnittliche Teilchengröße, bestimmt mittels Laserdiffraktometrie (LD) oder dynamischer Lichtstreuung (DLS) gemäß ISO 13320, von ≥ 50 nm bis ≤ 700 nm, noch weiter bevorzugt von ≥ 50 nm bis ≤ 400 nm auf.

Dadurch wird vorteilhafter Weise eine gute Handhabung für die Weiterverarbeitung in oralen und parenteralen Darreichungsformen erreicht.

Bevorzugt weisen die ausgefällten Nanoteilchen dabei einen D(v)90-Wert von ≤ 800 nm, besonders bevorzugt von ≤ 700 nm, insbesondere ≤ 650 nm auf. Unter dem D(v)90-Wert wird dabei verstanden, dass die Teilchen, welche einen Durchmesser kleiner oder gleich dem D(v)90-Wert aufweisen, 90% des Teilchenvolumens ausmachen.

Bevorzugt weisen die ausgefällten Nanoteilchen dabei einen D(v)50-Wert von ≤ 500 nm, besonders bevorzugt von ≤ 400 nm, insbesondere ≤ 350 nm auf. Unter dem D(v)50-Wert wird dabei verstanden, dass die Teilchen, welche einen Durchmesser kleiner oder gleich dem D(v)50-Wert aufweisen, 50% des Teilchenvolumens ausmachen.

Bevorzugt weisen die ausgefällten Nanoteilchen dabei einen D(v)10-Wert von ≥ 20 nm, besonders bevorzugt von ≥ 50 nm, insbesondere ≤ 150 nm auf. Unter dem D(v)10-Wert wird dabei verstanden, dass die Teilchen, welche einen Durchmesser kleiner oder gleich dem D(v)10-Wert aufweisen, 10% des Teilchenvolumens ausmachen.

In einer Ausgestaltung der Erfindung kann vorgesehen sein, dass der Volumenstrom des Lösungsmittels mit gelöstem Wirkstoff und der Volumenstrom des Anti-Lösungsmittels in einem Volumenverhältnis von Lösungsmittel zu Anti-Lösungsmittel im Bereich von ≥ 1:200 bis ≤ 2:1 zueinander liegen.

Vorzugsweise ist das Volumenverhältnis von Lösungsmittel zu Anti-Lösungsmittel im Bereich von ≥ 1:100 bis ≤ 1:1, mehr bevorzugt von ≥ 1:90 bis ≤ 1:5, beispielsweise 1:80, und besonders bevorzugt von ≥ 1:50 bis ≤ 1:5.

Bei dem Stabilisator handelt es sich um ein oder mehrere Additive, welche beispielsweise das Partikelwachstum hemmen, die Aggregation der Nanopartikel unterdrücken oder als Gerüstbildner die Redispergierbarkeit der Nanopartikel verbessern. Die Zugabe des Stabilisators kann bevorzugt im Solvent und/oder im Antisolvent erfolgen. Die Wahl des Stabilisators oder der Stabilisatoren sind von der Stabilisator-Solvent bzw. Stabilisator-Antisolvent-Wechselwirkung abhängig. Stabilisatoren schließen beispielsweise Polymere, Copolymere, Polyelektrolyte, Metallsalze und ionische und nicht-ionische Tenside, sowie starke Ionen mit ein. Besonders bevorzugt werden kleine Stabilisator-Moleküle mit einer hohen Diffusionsgeschwindigkeit bzw. Mobilität bevorzugt, um die durch schnelle Vermischung von Solvent und Antisolvent ausgefällten Partikeloberflächen zu stabilisieren.

Die Konzentration der Stabilisatoren hängt von der Rivaroxabankonzentration oder dem Gewichtsverhältnis von Rivaroxaban zu Stabilisator in der Suspension ab. Das Gewichtsverhältnis von Rivaroxaban zu Stabilisator in der Suspension liegt dabei vorzugsweise im Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1.

In einer bevorzugten Ausgestaltung der Erfindung kann vorgesehen sein, dass der Stabilisator mindestens ein ionisches Tensid aufweist.

Das ionische Tensid setzt die Oberflächenspannung herab und ermöglicht eine schnelle Vermischung von Solvent und Antisolvent und reduziert gleichzeitig auch die Ostwaldreifung.

Dadurch kann eine verbesserte Redispergierbarkeit der Aggregate nach Schritt c) erreicht werden.

Das ionische Tensid kann ein anionisches, kationisches oder zwitterionisches (amphoteres) Tensid sein.

In einer weiteren Ausführungsform ist das ionische Tensid ausgewählt aus:
Acylaminosäuren (und deren Salze), wie: Acylglutamate, beispielsweise Natriumacylglutamat, Di-TEA-palmitoylaspartat und Natriucaprylglutamat; Acylpeptide, beispielsweise Palmitoylhydrolysiertes Milchprotein, Natrium Cocoylhydrolysiertes Soja Protein und Natrium-/Kalium Cocoyl-hydrolysiertes Kollagen; Sarcosinate, beispielsweise Myristoyl Sarcosin, TEA-lauroyl Sarcosinat, Natriumlauroylsarcosinat und Natriumcocoylsarkosinat; Taurate, beispielsweise Natriumlauroyltaurat und Natriummethylcocoyltaurat; Acyllactylate, Luroyllactylat, Caproyllactylat, Alaninate; Carbonsäuren und Derivate, wie: Carbonsäuren, beispielsweise Laurinsäure, Aluminiumstearat, Magnesiumalkanolat und Zinkundecylenat, Ester-Carbonsäuren, beispielsweise Calciumstearoyllactylat und Natrium PEG-Lauramidcarboxylat, EtherCarbonsäuren, beispielsweise Natriumlaureth-Carboxylat und Natrium PEG-Cocamide Carboxylat; Phosphorsäureester und Salze, wie beispielsweise DEA-Oleth--Phosphat und Dilaureth-Phosphat; Sulfonsäuren und Salze, wie Acyl-isethionate, z.B. Natrium-/ Ammoniumcocoyl-isethionat, Alkylarylsulfonate, Alkylsulfonate, beispielsweise Natriumcocosmonoglyceridsulfat, Natrium C-Olefin-sulfonat, Natriumlaurylsulfoacetat und Magnesium PEG-Cocamidsulfat, Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat; sowie Schwefelsäureester, wie Alkylethersulfat, beispielsweise Natrium-, Ammonium-, Magnesium-, MIPA-, TIPA-Laurethsulfat, Natriummyrethsulfat und Natrium C-Parethsulfat, Alkylsulfate, beispielsweise Natrium-, Ammonium-und TEA-Laurylsulfat.

Erfindungsgemäß können das oder die ionischen Tenside ferner vorteilhaft gewählt werden aus der Gruppe der kationischen Tenside. Vorteilhaft zu verwendende kationische Tenside sind Alkylamine, Alkylimidazole, ethoxylierte Amine, quaternäre Tenside und Esterquats.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder - bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersultate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Erfindungsgemäß können das oder die ionischen Tenside vorteilhaft gewählt werden aus der Gruppe der amphoteren Tenside.

Vorteilhaft zu verwendende amphotere Tenside sind: Acyl-/dialkylethylendiamine, beispielsweise Natriumacylamphoacetat, Dinatriumacylamphodipropionat, Dinatriumalkylamphodiacetat, Natriumacylamphohydroxypropylsultonat, Dinatriumacylamphodiacetat und Natriumacylamphopropionat sowie N-Alkylaminosäuren, beispielsweise Aminopropylalkylglutamid, Alkylaminopropionsäure, Natuiumalkylimidodipropionat und Lauroamphocarboxyglycinat.

Bevorzugt als Tensid ist Natriumdodecylsulfat (SDS), Natriumdocusat (Dioctylnatriumsulfosuccinat), Natriumoleat und/oder Natriumdesoxycholat.

Das Tensid ist dabei vorzugsweise im Anti-Lösungsmittel gelöst, wobei die Konzentration des Tensids im Anti-Lösungsmittel so gewählt ist, dass das Gewichtsverhältnis des Tensids in der Suspension von Schritt b) zum Rivaroxaban in der Suspension von Schritt b) in einem Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1 liegt.

Dabei ist die Gewichtskonzentration des Tensids in der Suspension von Schritt b) abhängig von der Konzentration des Tensids im Anti-Lösungsmittel und dem Volumenverhältnis von Lösungsmittel zu Anti-Lösungsmittel.

Das Tensid ist dabei beispielsweise in einer Konzentration im Bereich von ≥ 0.2 mg/ml bis ≤ 2 mg/ml im Anti-Lösungsmittel gelöst.

In einer bevorzugten Ausgestaltung der Erfindung kann vorgesehen sein, dass das ionische Tensid im Anti-Lösungsmittel gelöst ist und im Wesentlichen Dioctylnatriumsulfosuccinat aufweist, wobei die Konzentration des Dioctylnatriumsulfosuccinat im Anti-Lösungsmittel so gewählt ist, dass das Gewichtsverhältnis des Tensids in der Suspension von Schritt b) zum Rivaroxaban in der Suspension von Schritt b) in einem Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1 liegt.

In einer bevorzugten Ausgestaltung der Erfindung kann vorgesehen sein, dass das ionische Tensid im Wesentlichen Dioctylnatriumsulfosuccinat aufweist und bevorzugt in einer Konzentration im Bereich von ≥ 0.2 mg/ml bis ≤ 2 mg/ml im Anti-Lösungsmittel gelöst vorliegt.

Besonders bevorzugt ist das Tensid dabei in einer Konzentration im Bereich von ≥ 0.2 mg/ml bis ≤ 1 mg/ml, insbesondere ≥ 0.5 mg/ml bis ≤ 0.6 mg/ml im Anti-Lösungsmittel gelöst.

In einer bevorzugten Ausgestaltung der Erfindung kann vorgesehen sein, dass der Stabilisator ein wasserlösliches Polymer aufweist.

Unter "wasserlöslich" ist hierbei zu verstehen, dass sich bei 20 °C in 100 g Wasser mindestens 0.5 g, bevorzugt mindestens 2 g des Polymers auflösen beziehungsweise unter Gelbildung lösen.

Das Polymer kann ausgewählt sein aus der Gruppe: Alkylcellulosen, Hydroxyalkylcellulosen, Hydroxyalkylalkylcellulosen, Carboxyalkylcellulosen, Alkalimetallsalze der Carboxyalkylcellulosen, Carboxyalkylalkylcellulosen, Carboxyalkylcelluloseester, Stärken, Pektine, Chitinderivate, Polysaccharide, Polyacrylsäure und ihre Salze, Polymethacrylsäure und ihre Salze, Polyvinylalkohol, Polyvinylpyrrolidon, Polyalkylenoxide oder eine Mischung aus mindestens zwei der vorgenannten Polymere.

Vorzugsweise ist das Polymer ausgewählt aus: Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Natriumcarboxymethylcellulose, Carboxymethylethylcellulose, Carboxyalkylcelluloseester, Stärken, Natriumcarboxymethylamylopektin, Chitosan, Dextransulfat Natriumsalz, Alginsäure, Alkalimetall- und Ammoniumsalze der Alginsäure, Carrageenane, Galaktomannane, Traganth, Agar-Agar, Gummi arabicum, Guargummi, Xanthangummi, Polyacrylsäure und ihre Salze, Polymethacrylsäure und ihre Salze, Polyvinylalkohol, Polyvinylpyrrolidon, Polyethylenoxid, Polypropylenoxid, Copolymere aus Ethylenoxid und Propylenoxid, N-Vinylpyrrolidon-VinylacetatCopolymere oder eine Mischung aus mindestens zwei der vorgenannten Polymere. Besonders bevorzugt sind Polyvinylpyrrolidone (insbesondere K12 und K30-Typen) und N-Vinylpyrrolidon-Vinylacetat-Copolymere. Bevorzugt kann das Polymer ein Copolymer aus Ethylenoxid und Propylenoxid sein, insbesondere ein Poloxamer, oder ein Polyvinylpyrrolidon, insbesondere PVPK30.

Das Polymer ist dabei vorzugsweise im Lösungsmittel gelöst, wobei die Konzentration des Polymers im Lösungsmittel so gewählt ist, dass das Gewichtsverhältnis des Polymers im Lösungsmittel zum Rivaroxaban im Lösungsmittel in einem Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1 liegt.

Das Polymer ist dabei beispielsweise in einer Konzentration im Bereich von ≥ 10 mg/ml bis zur Löslichkeitsgrenze im Lösungsmittel gelöst.

In einer bevorzugten Ausgestaltung der Erfindung kann vorgesehen sein, dass das wasserlösliche Polymer im Lösungsmittel gelöst ist und im Wesentlichen Poloxamer 188 oder PVPK30 aufweist, wobei die Konzentration des Polymers im Lösungsmittel so gewählt ist, dass das Gewichtsverhältnis des Polymers im Lösungsmittel zum Rivaroxaban im Lösungsmittel in einem Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1 liegt.

In einer bevorzugten Ausgestaltung der Erfindung kann vorgesehen sein, dass das wasserlösliche Polymer im Wesentlichen Poloxamer 188 oder PVPK30 aufweist und bevorzugt in einer Konzentration im Bereich von ≥ 10 mg/ml bis ≤ 100 mg/ml im Lösungsmittel gelöst vorliegt.

Besonders bevorzugt ist das Polymer dabei in einer Konzentration im Bereich von ≥ 40 mg/ml bis ≤ 50 mg/ml im Lösungsmittel gelöst.

Das Polymer kann vorzugsweise im Lösungsmittel und im Anti-Lösungsmittel gelöst sein, wobei die Konzentration des Polymers im Lösungsmittel so gewählt ist, dass das Gewichtsverhältnis des Polymers im Lösungsmittel zum Rivaroxaban im Lösungsmittel in einem Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1 liegt und die Konzentration des Polymers im Anti-Lösungsmittel so gewählt ist, dass das Gewichtsverhältnis des Polymers im Anti-Lösungsmittel zum Rivaroxaban im Lösungsmittel in einem Bereich von ≥ 1:100 bis ≤ 100:1, bevorzugt von ≥ 1:50 bis ≤ 50:1, besonders bevorzugt von ≥ 1:10 bis ≤ 10:1, beispielsweise bei 1:5 liegt.

Das Polymer ist dabei beispielsweise in einer Konzentration im Bereich von 1 mg/ml(...) bis zur Löslichkeitsgrenze im Anti-Lösungsmittel gelöst.

In einer bevorzugten Ausgestaltung der Erfindung kann vorgesehen sein, dass das wasserlösliche Polymer im Lösungsmittel und im Anti-Lösungsmittel gelöst ist und im Wesentlichen Poloxamer 188 oder PVPK30 aufweist, wobei die Konzentration des Polymers im Lösungsmittel so gewählt ist, dass das Gewichtsverhältnis des Polymers im Lösungsmittel zum Rivaroxaban im Lösungsmittel in einem Bereich von ≥ 1:100 bis ≤ 100:1, bevorzugt von ≥ 1:10 bis ≤ 10:1, besonders bevorzugt von ≥ 1:1.1 bis ≤ 1.1:1, beispielsweise bei 1:1 liegt und die Konzentration des Polymers im Anti-Lösungsmittel so gewählt ist, dass das Gewichtsverhältnis des Polymers im Anti-Lösungsmittel zum Rivaroxaban im Lösungsmittel in einem Bereich von ≥ 1:100 bis ≤ 100:1, bevorzugt von ≥ 1:50 bis ≤ 50:1, besonders bevorzugt von ≥ 1:10 bis ≤ 10:1, beispielsweise bei 1:5 liegt.

In einer bevorzugten Ausgestaltung der Erfindung kann vorgesehen sein, dass das wasserlösliche Polymer im Wesentlichen Poloxamer 188 oder PVPK30 aufweist und bevorzugt in einer Konzentration im Bereich von ≥ 10 mg/ml bis ≤ 100 mg/ml im Lösungsmittel und in einer Konzentration von 1 mg/ml bis 10 mg/ml im Anti-Lösungsmittel gelöst vorliegt.

Besonders bevorzugt ist das Polymer dabei in einer Konzentration im Bereich von ≥ 40 mg/ml bis ≤ 50 mg/ml im Lösungsmittel und in einer Konzentration von 1 mg/ml bis 10 mg/ml im Anti-Lösungsmittel gelöst.

In einer bevorzugten Ausgestaltung der Erfindung kann vorgesehen sein, dass der Stabilisator ein Tensid und ein Polymer aufweist.

Ohne auf eine Theorie festgelegt zu sein wird angenommen, dass das ionische Tensid sich insbesondere in Kombination mit einem Polymer positiv auf die Stabilität der Rivaroxabanpartikel während schritt c) auswirkt. Die Partikel sind folglich durch die Kombination aus elektrostatischer und sterischer Stabilisierung leichter redispergierbar.

Vorzugsweise kann ein Gewichtsverhältnis von Rivaroxaban: Polymer : Tensid in der Suspension von Schritt b) von ≥ 0.1 bis ≤ 5 : 1 : ≥ 0.1 bis ≤ 5 vorliegen, bevorzugt von ≥ 0.5 bis ≤ 1.5 : 1 : ≥ 0.5 bis ≤ 1.5.

In einer bevorzugten Ausgestaltung der Erfindung kann vorgesehen sein, dass in Schritt a) zusätzlich ein Peptisator bereitgestellt wird, wobei der Peptisator in dem Lösungsmittel und/oder dem Anti-Lösungsmittel gelöst vorliegt.

Unter Peptisatoren sind stark geladener Ionen zu verstehen, beispielsweise aus den wasserlöslichen Salzen Kaliumtartrat, Natriumoxalat, Calciumcitrat, Natriumpyrophosphat und Natriumcitrat, die zu einer hohen Partikelladung beitragen.

Der Peptisator ist dabei vorzugsweise im Anti-Lösungsmittel gelöst, wobei die Konzentration des Peptisators im Anti-Lösungsmittel so gewählt ist, dass das Gewichtsverhältnis des Peptisators in der Suspension von Schritt b) zum Rivaroxaban in der Suspension von Schritt b) in einem Bereich von ≥ 1:100 bis ≤ 100:1, bevorzugt von ≥ 1:10 bis ≤ 10:1, besonders bevorzugt von ≥ 2:1.1 bis ≤ 2.1:1, beispielsweise bei 2:1 liegt.

Dabei ist die Gewichtskonzentration des Peptisators in der Suspension von Schritt b) abhängig von der Konzentration des Peptisators im Anti-Lösungsmittel und dem Volumenverhältnis von Lösungsmittel zu Anti-Lösungsmittel.

Der Peptisator ist dabei beispielsweise in einer Konzentration im Bereich von ≥ 0.4 mg/ml bis ≤ 4 mg/ml im Anti-Lösungsmittel gelöst.

In einer Ausgestaltung der Erfindung kann vorgesehen sein, dass der Peptisator im Anti-Lösungsmittel gelöst ist und im Wesentlichen Natriumcitrat aufweist, wobei die Konzentration des Peptisators im Anti-Lösungsmittel so gewählt ist, dass das Gewichtsverhältnis des Peptisators in der Suspension von Schritt b) zum Rivaroxaban in der Suspension von Schritt b) in einem Bereich von ≥ 1:100 bis ≤ 100:1, bevorzugt von ≥ 1:10 bis ≤ 10:1, besonders bevorzugt von ≥ 2:1.1 bis ≤ 2.1:1, beispielsweise bei 2:1 liegt.

In einer Ausgestaltung der Erfindung kann vorgesehen sein, dass der Peptisator im Wesentlichen Natriumcitrat aufweist und bevorzugt in einer Konzentration im Bereich von ≥ 0.4 mg/ml bis ≤ 4 mg/ml im Anti-Lösungsmittel gelöst vorliegt.

Besonders bevorzugt ist der Peptisator dabei in einer Konzentration im Bereich von ≥ 0.4 mg/ml bis ≤ 4 mg/ml, insbesondere ≥ 1 mg/ml bis ≤ 1.2 mg/ml im Anti-Lösungsmittel gelöst.

In den Suspension in Schritt b) kann vorzugsweise ein Gewichtsverhältnis von Rivaroxaban : Polymer : Tensid : Peptisator von ≥ 0.5 bis ≤ 2 : 1 : ≥ 0.5 bis ≤ 2 : ≥ 1 bis ≤ 4 vorliegen, bevorzugt von ≥ 0.9 bis ≤ 1.1 : 1 : ≥ 0.9 bis ≤ 1.1 : ≥ 1.8 bis ≤ 2.2, beispielsweise von 1:1:1:2.

In einer Ausgestaltung der Erfindung kann das Lösungsmittel ein beliebiges organisches Lösemittel sein, welches Rivaroxaban adäquat löst. Das Lösemittel sollte mit dem Antisolvent mischbar sein. Bevorzugt weist das ausgewählte Solvent ideales Mischverhalten mit dem Antisolvent auf, sodass die Lösung verzögerungsfrei in der Partikelsuspension verteilt wird.

In einer weiteren Ausführungsform des Verfahrens kann das Lösungsmittel protisch sein, beispielsweise ein Alkanol. Beispiele für Alkanole sind Methanol, Ethanol, Isopropanol, n-Propanol. In einer weiteren Ausführungsform des Verfahrens kann das Lösungsmittel aprotisch sein. Beispiele für aprotische Lösungsmittel sind THF, DMSO, DMF und NMP. Das Lösungsmittel kann auch Mischungen aus wenigstens zwei der vorgenannten Substanzen aufweisen.

In einer Ausgestaltung der Erfindung kann vorgesehen sein, dass das Lösungsmittel Dimethylformamid ist.

In einer bevorzugten Ausgestaltung der Erfindung kann vorgesehen sein, dass das Anti-Lösungsmittel Wasser ist.

In einer besonders bevorzugten Ausgestaltung der Erfindung kann vorgesehen sein, dass das Lösungsmittel Dimethylformamid ist und das Anti-Lösungsmittel Wasser ist.

Es kann zudem vorgesehen sein, dass die Temperatur des Lösungsmittels und/oder das Anti-Lösungsmittels eingestellt wird.

Das Lösungsmittel kann dabei vorzugsweise auf eine Temperatur eingestellt werden, die es erlaubt die Löslichkeit des Rivaroxabans im Lösungsmittel zu vergrößern. Bevorzugt weist das Lösungsmittel eine Temperatur im Bereich von ≥ 15 °C bis ≤ 30 °C, besonders bevorzugt von ≥ 18 °C bis ≤ 25 °C, insbesondere von ≥ 19 °C bis ≤ 21 °C auf.

Das Anti-Lösungsmittel kann dabei vorzugsweise auf eine Temperatur eingestellt werden, die es erlaubt die Löslichkeit des Rivaroxabans im Anti-Lösungsmittel zu verkleinern. Bevorzugt weist das Anti-Lösungsmittel eine Temperatur im Bereich von ≥ 0 °C bis ≤ 15 °C, besonders bevorzugt von ≥ 1 °C bis ≤ 10 °C, insbesondere von ≥ 1 °C bis ≤ 5 °C auf.

In einer bevorzugten Ausgestaltung der Erfindung kann vorgesehen sein, dass der Mikromischer einen Ultraschallmischer aufweist.

In einer alternativen Ausgestaltung der Erfindung kann vorgesehen sein, dass vor Schritt c) die Suspension aus Schritt b) mit Ultraschall behandelt wird, vorzugsweise in einem Ultraschallbad.

Eine Ultraschallbehandlung im Ultraschallbad kann dabei bei einer Temperatur in einem Bereich von ≥ 0 °C bis ≤ 5 °C, insbesondere bei ≤ 2 °C, für eine Dauer in einem Bereich von ≥ 15 s bis ≤ 10 min, bevorzugt von ≥ 30 s bis ≤ 5 min, insbesondere von ≥ 1 min bis ≤ 3 min stattfinden.

Dadurch können vorteilhafter Weise besonders kleine Partikelgrößen erreicht werden.

In einer Ausgestaltung der Erfindung kann vorgesehen sein, dass zur Unterbindung des Partikelwachstums in Lösung und der Stabilisierung der Partikelgröße der Schritt c) vorzugsweise direkt nach der Fällung durchgeführt wird. Das Entfernen des Lösungsmittels und des Anti-Lösungsmittels kann auch eine verbesserte Handhabung bezüglich Schütt- und Fließfähigkeit der wirkstoffpartikelenthaltenden Feststoffe für die Weiterverarbeitung in oralen und parenteralen Darreichungsformen bieten.

In einer bevorzugten Ausgestaltung der Erfindung kann vorgesehen sein, dass Schritt c) ein Sprühtrocknungsschritt, ein Sprüh-Gefriertrocknungsschritt, ein Gefriertrocknungsschritt, ein Vakuumtrocknungsschritt oder ein Fallfilmverdampfungsschritt ist.

In einer weiteren Ausführungsform des Verfahrens wird nach Schritt b) die Suspension vom Mischer direkt in eine Sprühdüse eingeleitet und dort in Schritt c) versprüht und getrocknet.

In noch einer weiteren Ausführungsform des Verfahrens kann der Mischer direkt in einen Sprühtrockner im Sprühkopf eingebaut sein.

Die nach Schritt c) erhaltenen Aggregate des nanopartikulären Rivaroxabans können ganz oder teilweise auch als Aggregate des nanopartikulären Rivaroxabans eingebettet in einer Matrix des Stabilisators vorliegen.

In einer Ausführungsform des Verfahrens beinhaltet Schritt c) das Bereitstellen von Tröpfchen der Suspension und die Tröpfchen in Schritt c) weisen direkt nach ihrer Bereitstellung einen Durchmesser von ≥ 0.001 mm bis ≤ 3 mm auf. Bevorzugt ist ein Tröpfchendurchmesser von ≥ 0,001 mm bis ≤ 0,12 mm, mehr bevorzugt ≥ 0,001 mm bis ≤ 0,03 mm.

In einer weiteren Ausführungsform des Verfahrens weisen die erhaltenen Aggregate des nanopartikulären Rivaroxabans einen maximalen Durchmesser von ≥ 0.001 mm bis ≤ 1 mm auf. Bevorzugt ist ein Durchmesser von ≥ 0.001 mm bis ≤ 0,1 mm, mehr bevorzugt ≥ 0.001 mm bis ≤ 0,025 mm.

Optional, können Gerüstbildner im Solvent oder Anti-Solvent verwendet werden, sodass es nicht zum Kollaps der Aggregate bei Schritt c), insbesondere bei einer Gefriertrocknung kommt. Bevorzugt ist die Zugabe des Gerüstbildners im Antisolvent. Die Wahl des Gerüstbildners ist von der Gerüstbildner-Stabilisator-Solvent bzw. Gerüstbildner-Stabilisator-Antisolvent Wechselwirkung abhängen. Generell werden dem Fachmann bekannte kristalline Gerüstbildner, wie zum Beispiel Mannitol, oder Glycine, und amorphe Gerüstbildner, wie zum Beispiel Trehalose, Lactose, Sucrose, oder HPbCD verwendet. Der Gerüstbildner ermöglicht ein abfüllbares und handhabbares Prozessieren des getrockneten Materials.

Zur Herstellung einer galenischen Darreichungsform können die nach Schritt c) erhaltenen Aggregate des nanopartikulären Wirkstoffs wieder in einem Anti-Lösungsmittel redispergiert werden. Zur Beeinflussung des Redispergierverhaltens können Kombinationen aus Polymeren und ionischen Tensiden in verschiedenen Konzentrationsverhältnissen eingesetzt werden, um die Partikelgrößenverteilung (PGV) der Ursprungssuspension zu erhalten.

Bevorzugt ist, wenn die mittlere Partikelgröße (bestimmt mittels Laserdiffraktometrie (LD) oder dynamischer Lichtstreuung (DLS) gemäß ISO 13320) um nicht mehr als 500 % von der ursprünglichen mittleren Partikelgröße der gefällten Nanosuspension abweicht, bevorzugt < 100 %, und besonders bevorzugt ≤ 50 %, ganz besonders bevorzugt ≤ 10 %. Insbesondere wird eine Langzeitstabilität der redispergierten Nanopartikelsuspension im erfindungsgemäßen Verfahren ermöglicht. Bevorzugt liegt der D(90)-Wert der Partikelgrößenverteilung für die redispergierten Teilchen, bestimmt mittels dynamischer Lichtstreuung, unterhalb von 900 nm, weiter bevorzugt unterhalb von 600 nm und noch weiter bevorzugt unterhalb von 400 nm.

Das Gewichtsverhältnis von Rivaroxaban im gesamten Feststoffanteil nach der Redispergierung in Wasser kann bei bis zu 70 % liegen. Das Gewichtsverhältnis von Rivaroxaban im gesamten Feststoffanteil kann dabei im Bereich von 10 bis 60 % liegen, beispielsweise von 15 bis 25 %.

Mit der Erfindung wird ferner nanopartikuläres Rivaroxaban vorgeschlagen, welches eine durchschnittliche Teilchengröße, bestimmt mittels Laserdiffraktometrie (LD) oder dynamischer Lichtstreuung (DLS) gemäß ISO 13320, von ≥ 20 nm bis ≤ 900 nm aufweist.

Besonders bevorzugt weist das nanopartikuläre Rivaroxaban eine durchschnittliche Teilchengröße, bestimmt mittels Laserdiffraktometrie (LD) oder dynamischer Lichtstreuung (DLS) gemäß ISO 13320, von ≥ 50 nm bis ≤ 700 nm, noch weiter bevorzugt von ≥ 100 nm bis ≤ 400 nm auf.

Das nanopartikuläre Rivaroxaban kann dabei insbesondere in einer Suspension vorliegen, aufweisend Stabilisator und optional Peptisator.

Mit der Erfindung wird ferner eine Wirkstoffformulierung vorgeschlagen, welche zumindest nanopartikuläres Rivaroxaban, Natriumcitrat und Dioctylnatriumsulfosuccinat aufweist.

Die Wirkstoffformulierung kann dabei vorzugsweise mit dem vorbeschriebenen Verfahren nach Schritt c) erhalten werden. Dabei kann die Wirkstoffformulierung geeignet sein, um in einem Anti-Lösungsmittel redispergiert zu werden, wobei insbesondere eine Langzeitstabilität ermöglicht wird.

Die Erfindung wird nachfolgend anhand von Figuren und Beispielen weiter beschrieben.

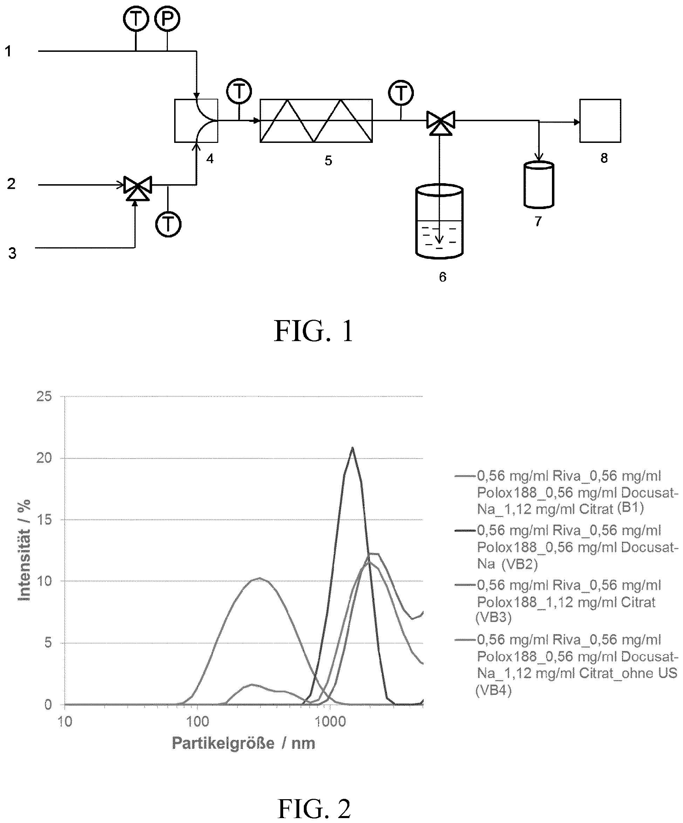

FIG. 1 ist eine schematische Darstellung des Prozesses in der vorliegenden Erfindung und stellt einen kontinuierlichen Solvent-Antisolvent Fällungsprozess dar. Der Prozess beinhaltet den Solvent Feed 1 und Antisolvent Feed 2, welche im Mikromischer 4 ideal vermischt werden, danach strömt die Mischung in den Mikrokanalreaktor 5, in der die Keimbildung und Wachstum zu Mikro- oder Nanopartikel stattfindet. Die Partikelsuspensionen werden in den Probenbehälter 7 aufgefangen um die Partikelgröße zu messen, bis die Partikelgröße oder Qualität den Anforderungen entspricht, falls nicht, wird die Suspension in den Abfallbehälter 6 geführt. Die Suspensionen werden dann nach unterschiedlichen Methoden behandelt entsprechend der Wirkstoff Darreichungsform.

Zum Beispiel, falls die Verabreichungsform das Rivaroxaban in Pulverform verlangt, wird die Nanopartikelsuspension in den Nachbehandlungsschritt 8 geführt, wie der Gefriertrocknung, Sprühtrocknung, Sprüh-Gefriertrocknung, Rotationsverdampfung oder Fallfilmverdampfung um das Lösemittel zu entfernen und das getrocknete Nanopartikelpulver zu erhalten.

Falls die Zuführungsform eine Nanopartikelsuspension ist, wird die ursprüngliche Partikelsuspension weiter verdünnt oder aufkonzentriert, um die geeignete Konzentration an Wirkstoff in der Suspension zu erhalten. Optional ist Reinigungssolvent Feed 3 eingeplant, um die Wirkstoffpartikeln im Prozess aufzulösen, falls die Partikeln den Mikrokanalreaktor 5 verblocken oder die Nebenanlagenteile verblocken.

### Beispiele: Fällung von Rivaroxaban Nanopartikeln

### Beispiel 1 (Fig. 2):

Reinstwasser (Widerstandswert > 18,2 MOhm cm) wurde als Antisolvent im Prozess genutzt. Als Solvent wurde Dimethylformamid (DMF) verwendet. Zur Förderung der Solvent und Antisolvent-Lösung wurden HPLC Pumpen mit konstantem Volumenstrom genutzt. Der Solvent-Feed wurde auf 20 °C temperiert und der Antisolvent Feed auf < 5 °C, sodass die Produkttemperatur bei < 5 °C lag. Im Solvent wurde Rivaroxaban, als Wirkstoff und Poloxamer 188 als Stabilisator gelöst. Die Wirkstoff- und Stabilisator-Konzentration lag bei 45 mg/ml und damit bei einer 1:1 Mischung. Im Antisolvent wurde 1,12 mg/ml Natriumcitrat und 0,56 mg/ml Natriumdioctylsulfosuccinat (Docusat-Natrium) verwendet. Der Antisolvent-Feed wurde bei 80 ml/min gefördert, während der Solvent-Feed bei 1 ml/min lag. Im Mischer wurde eine Nanopartikelsuspension geformt. Die Nanosuspension beinhaltet 0,56 mg/ml Rivaroxaban, 0,56 mg/ml Poloxamer 188, 0,56 mg/ml Docusat-Natrium und 1,12 mg/ml Natriumcitrat. Die Nanosuspension wurde direkt nach der Fällung für 2 min in einem 2 °C kalten Eisbad Ultraschall (US) behandelt. Die Partikelgröße wurde mittels Dynamischer Lichtstreuung (Malvern Nano Serie) ohne Filtration gemessen (siehe Beispiel 1, B1, FIG. 2). Die D(v)10-Werte lagen bei 149 nm, die D(v)50-Werte bei 338 nm und die D(v)90-Werte bei 626 nm.

Vergleichsweise wurden in der Formulierung das Docusat-Natrium (Verbleichsbeispiel 2, VB2) oder das Natriumcitrat (Vergleichsbeispiel 3, VB3) entfernt und bei gleichen Bedingungen wie in Beispiel 1 gefällt. Der D(v)90-Wert lag entsprechend bei 2350 nm (VB2) und 5590 nm (VB3). Weiter vergleichsweise wurde eine Nanosuspension mit 0,56 mg/ml Rivaroxaban, 0,56 mg/ml Poloxamer 188, 0,56 mg/ml Docusat-Natrium und 1,12 mg/ml Natriumcitrat ohne Nachbehandlung mit Ultraschall vermessen (Vergleichsbeispiel 3, VB4) und zeigte einen D(v)90-Wert von 3310 nm. Partikelgrößen nahe der Auflösungsgrenze der (Malvern Nano Serie) von 5000 nm weisen auf eine starke Polydispersität und breite Partikelgrößenverteilung hin.

FIG. 2 zeigt die Messwerte von parikulärem Rivaroxaban mit Poloxamer 188, Docusat-Natrium und Natriumcitrat (B1) im Vergleich zu den erhaltenen Partikeln nach Weglassen von Docusat-Natrium (VB2) oder Natriumcitrat (VB3), beziehungsweise ohne Ultraschallnachbehandlung (VB4).

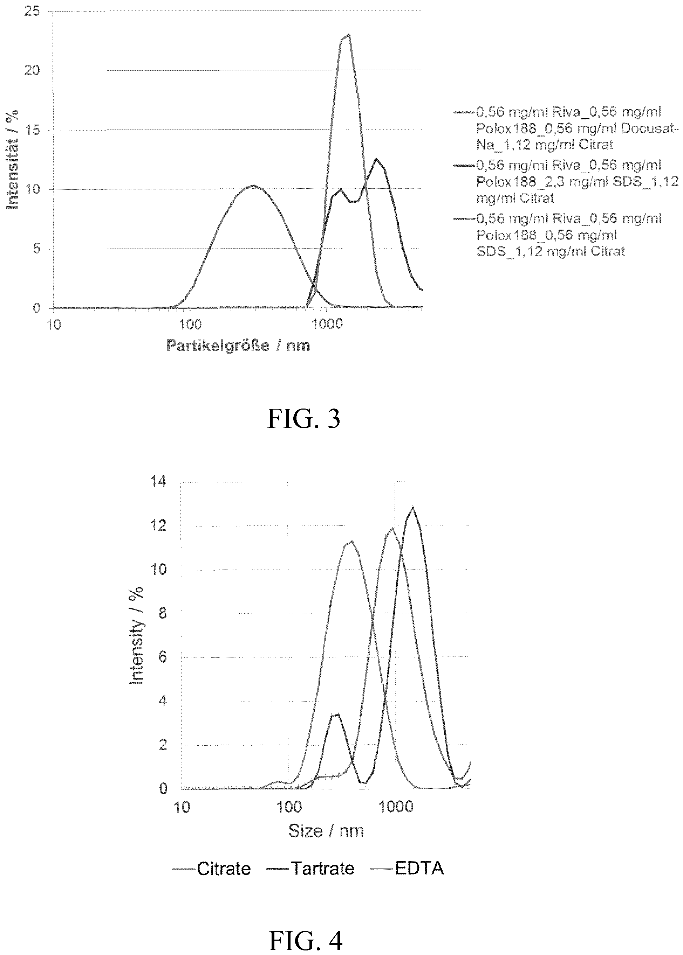

### Beispiel 2 (Fig. 3):

Die Nanosuspension wurde analog zu Beispiel 1 hergestellt. Als Solvent wurde Dimethylformamid (DMF) verwendet. Zur Förderung der Solvent und Antisolvent-Lösung wurden HPLC Pumpen mit konstantem Volumenstrom genutzt. Der Solvent-Feed wurde auf 20 °C temperiert und der Antisolvent Feed auf < 5 °C, sodass die Produkttemperatur bei < 5 °C lag. Im Solvent wurde Rivaroxaban, als Wirkstoff und Poloxamer 188 als Stabilisator gelöst. Die Wirkstoff- und Stabilisator-Konzentration lag bei 45 mg/ml und damit bei einer 1:1 Mischung. Im Antisolvent wurde 1,12 mg/ml Natriumcitrat und 0,56 mg/ml Natriumdioctylsulfosuccinat (Docusat-Natrium) bzw. 1,12 mg/ml Natriumcitrat mit 2,3 mg/ml SDS oder 0,56 mg/ml SDS verwendet.

### Beispiel 3 (Fig. 4):

Die Nanosuspension wurde analog zu Beispiel 1 hergestellt. Im Solvent wurde Rivaroxaban, als Wirkstoff und Poloxamer 188 als Stabilisator gelöst. Die Wirkstoff- und Stabilisator-Konzentration lag bei 45 mg/ml und damit bei einer 1:1 Mischung. Im Antisolvent wurde entweder als Peptisator 2,24 mg/ml Natriumcitrat oder 2,24 mg/ml Tartrat oder 2,24 mg/ml EDTA (basisches Millieu pH-Wert 12) und 0,56 mg/ml Natriumdioctylsulfosuccinat (Docusat-Natrium) hergestellt. Der Antisolvent-Feed wurde bei 80 ml/min gefördert, während der Solvent-Feed bei 2 ml/min lag. Im Mischer wird eine Nanopartikelsuspension geformt. Die Nanosuspension beinhaltet 1,12 mg/ml Rivaroxaban, 1,12 mg/ml Poloxamer 188, 1,12 mg/ml Docusat-Natrium und 2,24 mg/ml Peptisator. Die Nanosuspension wurde direkt nach der Fällung für 2 min in einem 2 °C kalten Eisbad Ultraschall (US) behandelt. Die Partikelgröße wurde mittels Dynamischer Lichtstreuung (Malvern Nano Serie) ohne Filtration gemessen (siehe Fig. 4).. Die D(v)10-Werte lagen bei 192 nm, die D(v)50-Werte bei 435 nm und die D(v)90-Werte bei 724 nm für die Formulierung mit Citrat. Der Wirkstoffgehalt konnte erfolgreich auf die doppelte Konzentration erhöht werden (siehe Beispiel 1). Die D(v)10-Werte lagen bei 246 nm, die D(v)50-Werte bei 639 nm und die D(v)90-Werte bei 2105 nm für die Formulierung mit Tartrat. Die D(v)10-Werte lagen bei 436 nm, die D(v)50-Werte bei 779 nm und die D(v)90-Werte bei 2000 nm für die Formulierung mit EDTA.

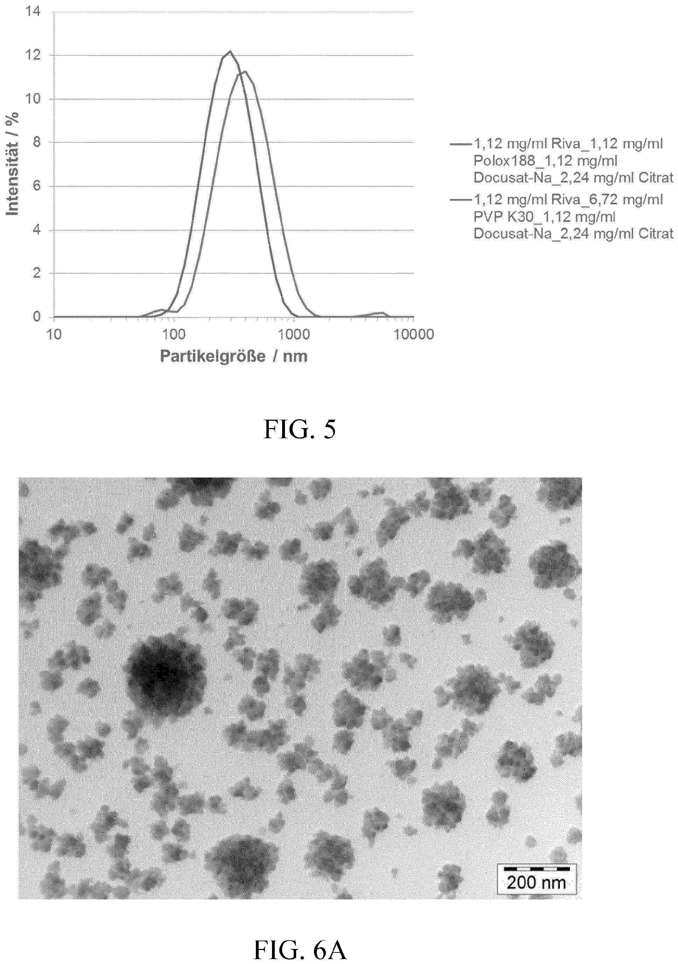

### Beispiel 4 (Fig. 5):

Die Nanosuspension wurde analog zu Beispiel 1 hergestellt. Im Solvent wurde Rivaroxaban, als Wirkstoff und PVP K30 als Stabilisator gelöst. Die Wirkstoff- und Stabilisator-Konzentration im Solvent lag bei 45 mg/ml und damit bei einer 1:1 Mischung. Im Antisolvent wurde 2,24 mg/ml Natriumcitrat, 1,12 mg/ml Natriumdioctylsulfosuccinat (Docusat-Natrium) und 5,6 mg/ml PVP K30 verwendet. Der Antisolvent-Feed wurde bei 80 ml/min gefördert, während der Solvent-Feed bei 2 ml/min lag. Im Mischer wird eine Nanopartikelsuspension geformt. Die Nanosuspension beinhaltet 1,12 mg/ml Rivaroxaban, 6,72 mg/ml PVP K30, 1,12 mg/ml Docusat-Natrium und 2,24 mg/ml Natriumcitrat. Die Nanosuspension wurde direkt nach der Fällung für 2 min in einem 2 °C kalten Eisbad Ultraschall (US) behandelt. Die Partikelgröße wurde mittels Dynamischer Lichtstreuung (Malvern Nano Serie) ohne Filtration gemessen. Die D(v)10-Werte lagen bei 162 nm, die D(v)50-Werte bei 330 nm und die D(v)90-Werte bei 572 nm.

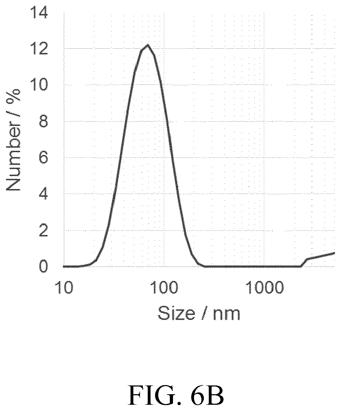

### Beispiel 5 (Fig. 6):

Die Nanosuspension aus Beispiel 4, welche 1,12 mg/ml Rivaroxaban, 6,72 mg/ml PVP K30, 1,12 mg/ml Docusat-Natrium und 2,24 mg/ml Natriumcitrat beinhaltet, wurde im Transmissionselektronenmikroskop untersucht. Agglomeratgrößen von 200 nm, aber vor allem auch Primärpartikelgrößen unter 100 nm sind dabei in der Suspension vorhanden (Fig. 6A). Die Anzahlverteilung aus der Dynamischer Lichtstreuung (Malvern Nano Serie) bestätigt zusätzlich das Ergebnis (Fig. 6B).

## Patentansprüche

1. Verfahren zur Herstellung nanopartikulären Rivaroxabans, umfassend die Schritte:
a) Bereitstellen einer Lösung von Rivaroxaban in einem Lösungsmittel, Bereitstellen eines Anti-Lösungsmittels für das Rivaroxaban und Bereitstellen von mindestens einem Stabilisator, wobei der Stabilisator in dem Lösungsmittel und/oder dem Anti-Lösungsmittel gelöst vorliegt und
b) Mischen der Lösung des Rivaroxabans in dem Lösungsmittel, des Antilösungsmittels und des Stabilisators in einem Mikromischer unter Erhalt einer Suspension aufweisend ausgefälltes Rivaroxaban, das Lösungsmittel und das Anti-Lösungsmittel,
wobei das ausgefällte Rivaroxaban in Form von Nanoteilchen vorliegt,
**dadurch gekennzeichnet, dass**
nach Schritt b) der Schritt c) durchgeführt wird:
c) Entfernen des Lösungsmittels und des Anti-Lösungsmittels insbesondere unter Erhalt von Aggregaten aufweisend das nanopartikuläre Rivaroxaban.

2. Verfahren gemäß Anspruch 1, wobei die in Schritt b) ausgefällten Nanoteilchen eine durchschnittliche Teilchengröße, bestimmt mittels Laserdiffraktometrie oder dynamischer Lichtstreuung gemäß ISO 13320, von ≥ 20 nm bis ≤ 900 nm aufweisen.

3. Verfahren gemäß einem der vorhergehenden Ansprüche, wobei der Volumenstrom des Lösungsmittels mit gelöstem Wirkstoff und der Volumenstrom des Anti-Lösungsmittels in einem Volumenverhältnis von Lösungsmittel zu Anti-Lösungsmittel im Bereich von ≥ 1:200 bis ≤ 2:1 zueinander liegen.

4. Verfahren gemäß einem der vorhergehenden Ansprüche, wobei der Stabilisator mindestens ein ionisches Tensid aufweist.

5. Verfahren gemäß Anspruch 4, wobei das ionische Tensid im Anti-Lösungsmittel gelöst ist und im Wesentlichen Dioctylnatriumsulfosuccinat aufweist, wobei die Konzentration des Dioctylnatriumsulfosuccinat im Anti-Lösungsmittel so gewählt ist, dass das Gewichtsverhältnis des Tensids in der Suspension von Schritt b) zum Rivaroxaban in der Suspension von Schritt b) in einem Bereich von ≥ 1:100 bis ≤ 100:1 liegt.

6. Verfahren gemäß einem der vorhergehenden Ansprüche, wobei der Stabilisator ein wasserlösliches Polymer aufweist.

7. Verfahren gemäß Anspruch 6, wobei das wasserlösliche Polymer im Lösungsmittel gelöst ist und im Wesentlichen Poloxamer 188 oder PVPK30 aufweist, wobei die Konzentration des Polymers im Lösungsmittel so gewählt ist, dass das Gewichtsverhältnis des Polymers im Lösungsmittel zum Rivaroxaban im Lösungsmittel in einem Bereich von ≥ 1:100 bis ≤ 100:1 liegt.

8. Verfahren gemäß einem der vorhergehenden Ansprüche, wobei in Schritt a) zusätzlich ein Peptisator bereitgestellt wird, wobei der Peptisator in dem Lösungsmittel und/oder dem Anti-Lösungsmittel gelöst vorliegt.

9. Verfahren gemäß Anspruch 8, wobei der Peptisator im Anti-Lösungsmittel gelöst ist und im Wesentlichen Natriumcitrat aufweist, wobei die Konzentration des Peptisators im Anti-Lösungsmittel so gewählt ist, dass das Gewichtsverhältnis des Peptisators in der Suspension von Schritt b) zum Rivaroxaban in der Suspension von Schritt b) in einem Bereich von ≥ 1:100 bis ≤ 100:1 liegt.

10. Verfahren gemäß einem der vorhergehenden Ansprüche, wobei das Lösungsmittel Dimethylformamid ist.

11. Verfahren gemäß einem der vorhergehenden Ansprüche, wobei das und das Anti-Lösungsmittel Wasser ist.

12. Verfahren gemäß einem der vorhergehenden Ansprüche, wobei vor Schritt c) die Suspension aus Schritt b) mit Ultraschall behandelt wird.

13. Verfahren gemäß einem der vorhergehenden Ansprüche, wobei Schritt c) ein Sprühtrocknungsschritt, ein Sprüh-Gefriertrocknungsschritt, ein Gefriertrocknungsschritt, ein Vakuumtrocknungsschritt oder ein Fallfilmverdampfungsschritt ist.

14. Nanopartikuläres Rivaroxaban, **dadurch gekennzeichnet, dass** das nanopartikuläre Rivaroxaban eine durchschnittliche Teilchengröße, bestimmt mittels Laserdiffraktometrie oder dynamischer Lichtstreuung gemäß ISO 13320, von ≥ 20 nm bis ≤ 900 nm aufweist.

15. Wirkstoffformulierung, zumindest aufweisend nanopartikuläres Rivaroxaban, Natriumcitrat und Dioctylnatriumsulfosuccinat.
